Europäisches Patentamt

European Patent Office

Office européen des brevets

(11) Publication number: **0 121 385**
**A1**

## EUROPEAN PATENT APPLICATION

(21) Application number: **84301987.8**

(22) Date of filing: **23.03.84**

(51) Int. Cl.³: **G 01 N 33/48**

(30) Priority: **26.03.83 GB 8308389**

(43) Date of publication of application:
**10.10.84 Bulletin 84/41**

(84) Designated Contracting States:
**AT BE CH DE FR GB IT LI NL SE**

(71) Applicant: **CAMBRIDGE LIFE SCIENCES PLC**
**Cambridge Science Park Milton Road**
**Cambridge CB4 4BH(GB)**

(72) Inventor: **Lowe, Christopher Robin**
**Windy Willows Potters Heron Close**
**Ampfield Romsey, S05 9BX(GB)**

(74) Representative: **Lambert, Hugh Richmond et al,**
**D. YOUNG & CO. 10 Staple Inn**
**London, WC1V 7RD(GB)**

(54) Conductimetric bioassay techniques.

(57) The application discloses a novel method and device for carrying out conductimetric bioassays of a given analyte in a liquid sample. For this purpose a probe is dipped into the sample comprising an insulating support (30) having mounted on its surface a porous membrane (56) onto which has been immobilised a specific binding partner, e.g. an enzyme, for the analyte. The resulting change of conductivity along the length of the membrane, which is proportional to the analyte concentration, is measured by means of electrodes (32, 34) embedded in the insulating support. A second inactive membrane (58) is preferably provided on the probe and contacted by a pair of identical reference electrodes (36, 38) and the conductivity measured on a comparative basis. Membrane materials are essentially electrically insulating materials such as cellulose acetate and cellulose nitrate. Any enzyme or ligand may be used which results in a change of conductivity along the length of the membrane as the result of the specific interaction with the analyte. Specific enzymes include pencillinase, urease, paracetamol, etc.

EP 0 121 385 A1

./...

Croydon Printing Company Ltd.

FIG.2

1

# CONDUCTIMETRIC BIOASSAY TECHNIQUES

## Field of Invention

This invention relates to conductimetric bioassay techniques and apparatus and conductimetric biosensors for use therein.

## Background and Prior Art

Various electrometric bioassay techniques using immobilised enzymes are known. For example, a variety of enzyme electrodes are known, particularly for use as glucose sensors, comprising a membrane containing an enzyme immobilised thereon and an electrode assembly for detecting amperometrically active species produced by the action of the enzyme on its respective substrate.

Included amongst such proposals are the enzyme electrodes disclosed in US-A-3,984,745 and EP 0 078 636. Such enzyme electrodes comprise a film or layer of immobilised enzyme on the face of an electrode. Such enzyme electrodes are dependent upon the production (or destruction) of an amperometrically active species, e.g. hydrogen peroxide, by the enzyme/substrate interaction, e.g. the oxidation of glucose by glucose oxidase, and the detection of that species by the measurements out of current output from electrode, and are not per se concerned with or dependent upon the electrical conductivity of the membrane.

This approach is also shown in US-A-4,240,889 and in US-A-4,307,195. In this case a polarographic cell is constructed comprising an anode, a cathode, and a semi-permeable membrane comprising an immobilised enzyme, e.g. glucose oxidase, defining a boundary wall of the polarographic cell and in face-to-face contact with the anode. When the cell is placed into a sample containing the enzyme susbtrate amperometrically active species resulting from the enzyme/substrate interaction permeate the membrane to affect the output current from the cell, which varies proportionally with the substrate concentration of the sample.

In DE-A-3 028 569 a laboratory technique is disclosed, which is said to be useful for a variety of purposes such as pH measurement, flow-rate measurement and temperature measurement, and which involves conductivity measurements made across the thickness of an immobilised enzyme membrane located in a conductivity cell between two calomel electrodes, the conductivity through the membrane being affected by an enzyme substrate dispersed as a mono-layer on the surface of the electrolyte and entrappable by a pin hole in the membrane as it is immersed in the electrolyte. This technique is, however, not concerned with, nor applicable to the measurement of substrate concentration and hence of no direct relevance to the conductimetric biossay technique of the present invention.

Another approach, specifically directed to the assay of the analyte and involving conductimetric measurement, rather than amperometric, is adopted in US-A-4,334,880. This utilizes the electrical characteristics of a specific binding substance, such as an antibody, antigen, enzyme or enzyme substrate immobilised on the surface of a semiconductor to modify the conductivity of the semiconductor, when in contact with the specific binding partner of that substance, the semiconductor in that case specifically being polyacetylene.In this case the change of conductivity of the semiconductor is used to measure the concentration of the analyte in the sample.

The present invention utilizes yet a different approach, namely detection and measurement of conductivity changes in an immoblised enzyme layer, or other specific binding partner for the analyte, and occuring as a result of the interaction between the specific binding partner and the analyte.

In accordance with this invention this is achieved by immobilising the enzyme or other specific binding partner for the analyte on a porous membrane, which itself is essentially non-conducting, i.e. having a conductivity below $10^{-9}$ Siemens/cm, but which in conjunction with the immobilised layer forms a member whose conductivity in the presence of the sample liquid containing the analyte, is affected by the interaction between the immobilised substance and the analyte in proportion to the concentration of the analyte. Thus by placing the membrane comprising the immobilised enzyme or other specific binding partner for the analyte in contact with the liquid medium containing the respective analyte, and measuring the resultant change of conductivity along the length of the membrane, the concentration of the analyte can be determined.

## Summary of the Invention

Thus, in one aspect the invention provides a conductimetric sensor for the quantitative bioassay of an analyte in a liquid sample comprising a sensing member whose conductivity, when in contact with the sample, is proportional to the concentration of the analyte in the sample, and a pair of spaced-apart electrodes in electrical contact with the sensing member and connectable to means for measuring the conductivity of the sensing member, wherein said sensing member comprises an essentially non conducting porous membrane with which the electrodes are in face-to-face electrical contact at two separate points spaced along the length the membrane, said membrane being formed from a material having a conductivity of less than $10^{-9}$ Siemens/cm, and having immobilised thereon a substance having a specific affinity for, or reactivity with the analyte, and reactive therewith in the presence of the sample to induce a change of conductivity along the length of the membrane between the two electrodes.

4

In a second aspect the invention provides a conductimetric bioassay method for the determination of an analyte in a liquid sample, which comprises contacting the sample with a sensor comprising an essentially non-conducting porous membrane formed from a material having a conductivity of less than $10^{-9}$ Siemens/cm and having immobilised thereon a substance having a specific affinity for or reactivity with the analyte, the interaction between the analyte and said substance in the presence of said sample being effective to cause a change in conductivity in the membrane, and measuring the change, if any, of conductivity of said membrane when in contact with said sample, said measurement being made along the length of the membrane.

Detailed Description

As the essentially non-conductive membrane for the immobilised enzyme, or other substance having a specific affinity for the analyte, any suitably inert synthetic plastics or naturally occuring insulating material may be used, such as polyethylene, polytetrafluoroethylene, polystyrene and other polyolefins, polyesters, polycarbonates and polyamides. Other suitable membrane materials include silicone rubber, cross-linked protein and collodion. Cellulosic membranes are, however, preferred, particularly methylcellulose, cellulose acetate and cellulose nitrate. Membrane thickness may range from 25 to 500 microns, and preferably from 50 to 250 microns and most preferably 100-200 microns. Desirably the membranes will have a pore size in the range 0.05 to 1 micron, preferably in the range 0.1 to 0.5 micron and a total pore volume in the range 50-90%, more usually and preferably in the range 72-80%. As will be appreciated, the membrane thickness and porosity will be inter-related and will be dependent upon a number of factors, e.g. physical integrity and strength, and also upon the particular analyte, it being a feature of the present invention that the measurement of conductivity along the length of the porous membrane incorporating the immobilised enzyme or other specific binding substance enables a high degree of selectivity to be achieved, which is not achieved by conductivity measurement carried out in free solution.

This can be particularly important in the assay of samples containing a plurality of conductive species, and where the interest is on only one of those species, i.e. the species produced or destroyed by the reaction between the specific binding partner and the analyte.

As the substance to be immobilised on the membrane a wide range of materials may be used including enzymes, haptens, antigens, antibodies or indeed any substance having a specific affinity for the analyte and which interacts therewith to induce a change of conductivity e.g. as the result of the generation or consumption of a charge species associated with the interaction between the substance and the analyte, or a change in mobility of a charged species, or a change in dipole moment of a dipole species. More particularly, however, the invention is directed to conductimetric enzymatic assays and for this purpose a wide variety of immobilised enzymes, which react with the analyte to cause the production of or destruction of a charged species, may be used. For example, immobilised enzymes suitable for use in this invention include oxidases, amidases, proteases, deaminases, hydrolases, nucleases, decarboxylases, kinases, phosphatases and lipase. Specific and preferred examples are penicillinase, urease, D-glucose oxidase, creatinases and creatininases such as creatinine amidohydrolase, creatinine iminohydrolase (creatinine desimidase) and creatine amidinohydrolase, pronase, L-asparaginase, alkaline phosphatase, sarcosin dehydrogenase, aryl acyl amylamidohydrolase (paracetamolase) and restriction endonuclease.

The methods used to immobilise the enzymes onto the membrane are those conventionally used in the art of enzyme immobilisation and need not be described in detail. Any such method which does not significantly affect the activity of the enzyme may be used, such as adsorption, encapsulation or cross-linking, which is the preferred method of immobilisaiton. A variety of cross-linking agents are available and

0121385

suitable for this purpose, one of the most convenient being glutaraldehyde which acts to cross link the enzyme to the membrane via amino groups in the enzyme.

In order to make the indicated conductivity measurements two electrodes will be placed into electrical contact with the membrane, preferably in face-to-face contact with the same face of the membrane, so that the conductivity measurements are made along the length of the membrane which provides a conductance member bridging the two electrodes. The spacing between the electrodes may range from 0.5 to 40 mm, more usually 1-10 mm, and most preferably 1-5 mm. The electrodes may either be in permanent electrical connection with the membrane or the connection may be purely temporary. A variety of electrically conductive materials, metals, alloys or even carbon may be employed as the electrode materials, but preferably they are of a biologically inert material e.g. gold or platinum.

Various configurations are possible for the conductimetric biosensors of the present invention, one of the most convenient being a probe or dipstick which can simply be immersed or dipped into the sample containing the analyte to be assayed. A particularly preferred probe configuration comprises an elongated insulating support member having a pair of electrodes, preferably gold or platinum, embedded therein and each having an electrode surface exposed adjacent the distal end of the probe. At that end the probe carries the membrane applied as a surface layer or film thereon and in face-to-face contact with the exposed surfaces of the two electrodes. Suitable leads will, of course, likewise, be embedded in the insulating support and extending along the length thereof to provide electrical contact with the electrodes. The membrane layer or film may be permanently adhered to the support, or carried by a membrane carrier detachably and interchangeably mounted on the support. In this way the membrane comprising the immobilised enzyme or other specific binding partner for the analyte can easily be replaced or exchanged for a different membrane having a different enzyme or specific binding partner immobilised thereon for the determination of a different analyte in the same or different sample.

The method of the invention may be used in two main ways:-

1.   Where the substance to be determined, i.e. the analyte, is the substrate of an enzyme catalysed reaction a single stage direct assay may be used.  For this purpose a sample with an unknown concentration of the substrate is contacted with a porous membrane comprising the immobilised enzyme.  The change in electrical conductivity as a result of the enzyme catalysed reaction is proportional to the amount of substrate in the sample, and the measured change in conductance is therefore proportional to the concentration of the substrate in the sample.

2.   Where the analyte is a component of a reaction between a ligand, for example, a hapten or antigen, and its corresponding specific binding partner, e.g. the corresponding antibody, which may be either poly- or monoclonal, the membrane does not initially have the enzyme associated therewith, but instead has either the ligand or specific binding partner immobilised in association therewith.  The assay is carried out as a two stage process:-

a)   The membrane with immobilised component is contacted with enzyme-labelled specific binding partner or ligand complementary to the immobilised component, and with a sample containing an unknown quantity of one of the components.  A competitive assay takes place, resulting in a certain number of enzyme-labelled components becoming associated with the membrane, the number depending on the quantity of unknown in the sample.

b)   The membrane with associated enzyme is contacted with substrate, resulting in a change in electrical conductance.  The measured change in conductance is proportional to the amount of enzyme associated with the membrane, which in turn is proportional to the amount of component to be measured in the sample.

With the two stage process, the order of addition of reactants is not important, but in general the sample and enzyme-labelled component will be added first, the membrane washed after equilibrium has been reached, and the substrate then added.

In the competitive assay of the first stage a), the component to be measured in the sample competes either:-

i)     with enzyme-labelled component for a limited number of complementary binding sites associated with the membrane;  or

ii)    with the component associated with the membrane for a limited number of complementary molecules in enzyme-labelled form.

In either case, the number of enzyme-labelled particles which become associated with the membrane, which is measured by adding substrate, and measuring the resultant change of conductivity along the length of the membrane in accordance with the technique of the invention provides an indication of the concentration of component in the sample.

The invention may thus be used not only for the determination of enzyme substrates but also for the determination of numerous ligands and specific binding partners, the former including trace metabolites such as steroids (e.g. cortisol, oestriol, testosterone, progesterone, $17\beta$-oestradiol, aldosterone etc.), therapeutic drugs (e.g. phenytoin, phenobarbital, genatmycin, propranolol, digoxin etc.), drugs of abuse (e.g. morphine, cocaine, amphetamine, barbituates, tetrahydrocannabinol etc.) thyroid function constituents (e.g. $T_4$, $T_3$, TBG, TSH etc.), protein hormones (e.g. insuling, growth hormones etc.), and proteins associated with pregnancy, cancer or other diagnosis (e.g. HPL, HCG, $\alpha$-feto-protein, pregnancy specific protein ($SP_1$)) and any other ligand or specific binding partner to which a conventional immunoassay procedure might not be applicable.

And in accordance with this latter aspect the invention also provides a conductimetric bioassay kit for the conductimetric assay of a ligand or a specific binding partner of a ligand in a sample, said kit comprising as a first item a porous membrane composed of a material having a conductivity of less than $10^{-9}$ Siemens/cm and having immobilized thereon a given quantity of a ligand or a specific binding partner of a ligand corresponding to the unknown, and as a second item, a given quantity of the unknown ligand or specific binding partner as the case may be, labelled with an enzyme, being such that when conjugated to the membrane by binding with the immobilized component, and contacted with an enzyme substrate, the conductivity of the conductive member formed by the membrane and the immobilised enzyme conjugate changes as a result of the enzyme/substrate interaction and proportional to the concentration of the unknown. As optional items such kits may also comprise a quantity of substrate for the enzyme and the means required to measure the conductivity of the membrane/enzyme conjugate.

Of course, where the immobilised component of the membrane itself is a hapten, or antigen or other ligand and where the interaction of that hapten or antigen with its specific binding partner is such as to produce a conductive species, or a change in concentration of a conductive species, then the concentration of the binding partner in a sample can be determined directly by the technique of the present invention without resorting to the competitive enzymatic technique described above.

For most purposes in accordance with this invention the conductivity measurements will be made on a comparative basis and to this end the conductimetric biosensors of this invention will usually comprise a second pair of reference electrodes identical with the first pair and likewise bridged by a membrane structure identical with the "active" membrane but modified only in that the immobilised substance is omitted, or at least, inactivated. The analyte concentrations are thus determined on the basis of the difference between the two conductivity measurements.

In a preferred technique, the conductivity measurements are alternately switched between the active and reference electrodes and the results displayed as a function of the cumulative totals of the successive differences in conductivity between the two pairs of electrodes measured over a predetermined period of time.

The invention is further illustrated by the accompanying drawings, in which:-

Figure 1 is a diagrammatic sketch illustrating the principle of the present invention;

Figure 2 is an illustration of a conductimetric biosensor in accordance with this invention in the form of a probe or dipstick;

Figures 3 and 4 are sections taken on lines III-III and IV-IV respectively of Figure 2;

Figure 5 shows an alternative form of probe with a detachable membrane carrier;

Figure 6 is an enlarged view of the membrane carrier;

Figures 7 and 8 are section taken on lines VII-VII and VIII-VIII respectively of Figure 5;

Figure 9 is a view similar to Figure 3 showing the use of the biosensor according to this invention for the biossay of analytes in samples, where the quantity of sample available for assay is extremely restricted; and

Figures 10-15 are graphs illustrating the results of conductimetric measurements using biosensors according to the invention.

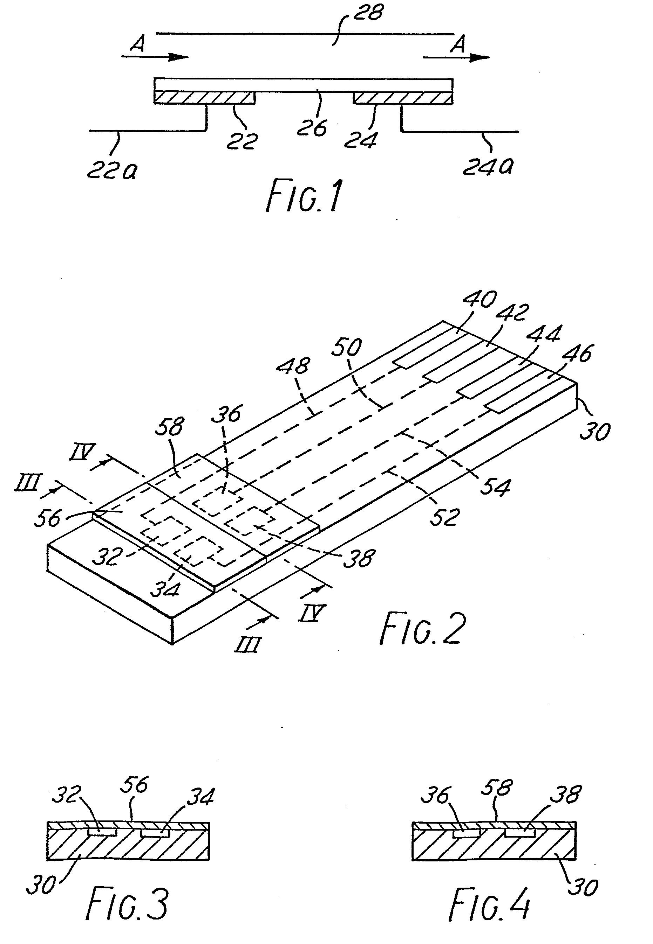

Referring to Figure 1 a schematic arrangement is shown for measuring the concentration of an analyte in a liquid medium A flowing through a conduit 28. Positioned in the conduit 28 in a position to be contacted by the liquid medium A is a porous membrane of electrically non-conductive material e.g. cellulose acetate or cellulose nitrate on which has been immobilised the enzyme or other substance having a specific affinity for the analyte and which interacts therewith to produce a change of conductivity along the length of the membrane 26 proportioned to the concentration of the analyte in the liquid medium A. This change of conductivity is measured by means of electrodes 22, 24, e.g. of gold or platinum, placed in face-toface electrical contact with the membrane, usually at a spacing of from 1-5 mm. Electrode size is immaterial provided that adequate electrical contact is obtained with the membrane. Typical sizes are 2 x 5 mm or larger, e.g. up to 0.5 x 1.0 cm.

As will be seen the membrane comprising the immobilised enzyme or other ligand specific to the analyte forms a conductance member spanning the electrodes 22, 24 and whose conductivity can be measured by conventional conductivity measuring apparatus (not shown) and techniques via electrical leads 22a and 24a to the two electrodes.

Referring to Figures 2-4 , these illustrate a conductimetric biosensor probe constructed in accordance with the principles of this invention. As shown, the probe comprises an elongated, rectangular section insulating support member 30 in which are embedded two identical pairs of gold electrodes 32, 34 and 36, 38. The electrodes are each approximately 1 x 4 mm in size and the two members of each pair are spaced approximately 1.5 mm apart. The upper surface of each electrode is exposed at the surface of the support adjacent one end, referred to herein as the distal end.

At the proximal end are four exposed terminals (40, 42, 44, 46) for connection to the conductivity measuring apparatus (not shown) and each connected to one of the electrodes by a lead (48, 50, 52, 54) wholly embedded in the support. As will be appreciated the support member comprising the electrodes can be readily fabricated using standard printed circuit techniques.

Applied to the surface of the support adjacent the distal end and forming a thin film or layer covering the two pairs of electrodes so as to be in electrical face-to-face contact therewith are two porous membrane structures (56, 58), e.g. a thin (150 microns) film of cellulose acetate or cellulose nitrate, the two membranes being identical save that one of the two has an enzyme or other specific binding partner for the analyte immobilised thereon, for example a penicillinase, urease, glucose oxidase, creatinase, creatininase, acid phosphatase etc., depending on the analyte to be determined. In order to make this determination, the distal end of the probe comprising the two membranes is introduced into the medium containing the analyte to be determined. The conductivities of the two membranes are then measured and a determination made of the concentration of the analyte based on the differential between the two values. Preferably this is done by alternate switching between the two membranes and the differential values obtained accumulated and stored for a predetermined time before presentation as a final cumulative total.

Conveniently the conductivity measurements are made using a 1 kHz sinusoidal excitation signal, which is switched at 0.5 second intervals between the "reference" pair of electrodes and the "test" pair of electrodes, to allow the differential conductivity to be determined within one second. Measurements are generally of the "stopped rate" type, in that an initial level is measured, having allowed the system to settle down (typically 15 seconds after introducing the substrate), and a final voltage change measured after a further 40 seconds. This voltage change is proportional to the total conductivity change over this period.

The instrumentation used converts the A.C. signals from the conductivity electrodes to D.C. levels (corresponding to the conductivity) by using precision rectifiers, and the D.C. levels are then stored by sample and hold circuits, allowing a differential amplifier to be used to provide the final voltage proportional to the differential conductivity.

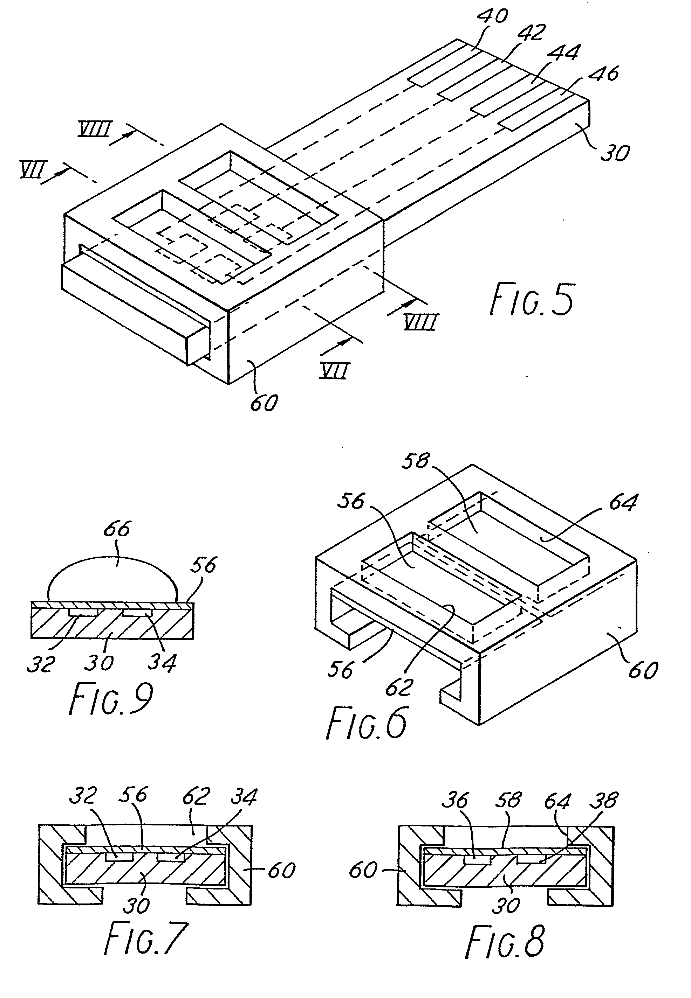

In the modification shown in Figures 5-8, the membranes (56, 58) are carried by a separate carrier (60) slidably and interchangeably received on the insulating support member. In its upper surface two apertures (62, 64) are provided to expose the membranes (56, 58) for contact with the sample when the probe is introduced therein. As will be appreciated this permits the same biosensor to be used for a wide range of assays, merely by changing the membrane and membrane carrier.

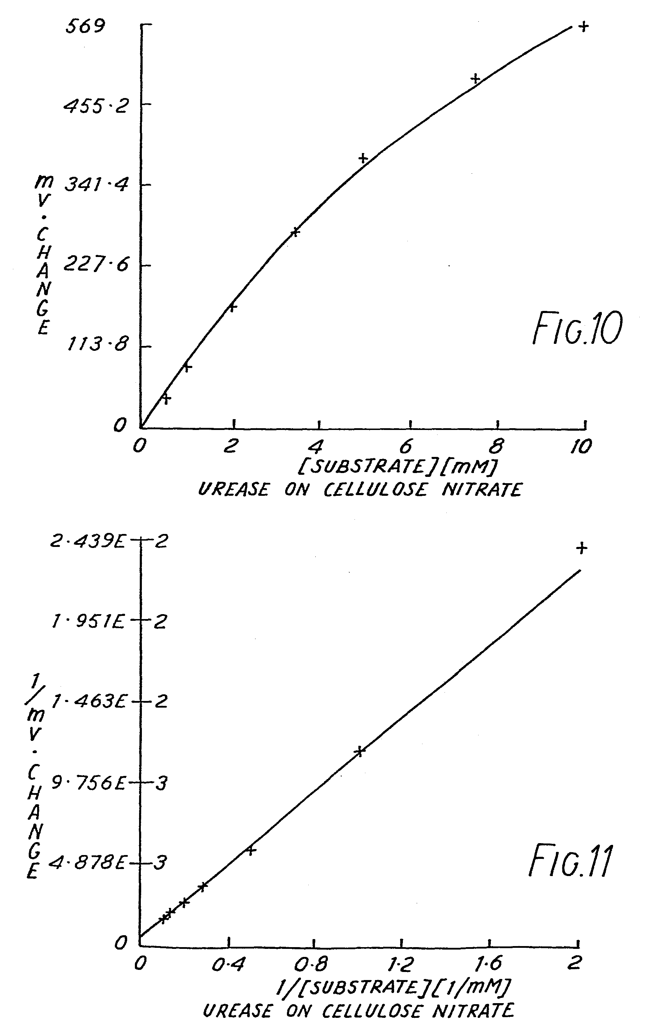

Whilst the invention has been so far described in terms of introducing the probe comprising the immobilised enzyme membrane into the sample, for example, by immersion therein, an important alternative technique for carrying out the method of this invention is shown in Figure 9. Here, instead of immersing the probe in the sample, just one or two drops (66) of the sample can be placed on the sensor, i.e. on the membranes (56,58), and the conductivity measurements made on the basis of that single drop. Thus, the method and apparatus of this invention enables assays to be carried out on extremely small samples, and this can be particularly important for diagnostic methods carried out on patient samples, which may indeed, be obtainable only in minute amounts. A considerable and distinct advantage is thus provided over conventional enzyme electrodes which required sample volumes of considerable size.

As has already been explained, the probes described above can be used either for the direct assay of an analyte, e.g. an enzyme substrate, or for the competitive assay of an unknown ligand. In the latter technique, the "active" membrane, in the first instance, will have immobilised thereon a specific binding partner for the unknown. This is then contacted with the unknown plus a known, restricted amount of the unknown conjugate with an enzyme, whereby competitive binding takes place with the immobilised binding partner. This results in the immobilisation of a restricted amount of the enzyme-lablled conjugate on the membrane, which amount is proportional to the unknown, and which can be determined by measurement of the enzymatic activity of the membrane by the technique of the present invention, i.e. by measurement of the conductivity of the membrane/enzyme conjugate when in the presence of the enzyme substrate.

The invention is illustrated by the following examples:

## EXAMPLE I

### Preparation of immobilised enzyme membranes

25 mm discs of cellulose nitrate membrane, thickness 150 microns, pore size 0.1 to 0.45 micron, total pore volume 72-80% were pre-soaked in 50 mM phosphate buffer, pH 6.5 at $4^{o}$C.

The pre-soaked discs were then transferred to 600 microlitres of the same buffer at $4^{o}$C additionally containing 3 mg. of urease, followed by 12 microlitres 25% glutaraldehyde solution. The mixture is then gently agitated at $4^{o}$C for 2 hours after which the membrane discs are washed to remove excess enzyme and stored for use at $4^{o}$C in 5 mM imidazole buffer pH 7.5.

By the same technique aryl acyl amylamidohydrolase (paracetamolase) has been immobilised onto cellulose nitrate, and penicillinase onto cellulose acetate. Other enzymes which can and have been immobilised by the same or similar techniques onto cellulose acetate and celluose nitrate membranes for use in the present invention include: glucose oxidase, asparaginase, pronase, carboxypeptidase G, creatine amidinohydrolase and creatinine amidohydrolase.

15

## EXAMPLE 2

Preparation of immobilised enzyme membranes

B. cereus pencillinase is coupled to a methyl cellulose membrane by initial activiation of the membrane with epichlorhydrin and subsequent reaction with 1,3-diamino-2-hydroxypropane. The immobilised residues are then coupled via the terminal amino groups to the pencillinase by cross-linking with glutaraldehyde. Amounts of immobilised enzyme up to 1.3 micrograms protein/gm. of moist membrane are obtained.

## EXAMPLE 3

Conductivity test procedures

For the purposes of these tests a probe was used substantially identical with that shown in Figure 2 using gold electrodes 1.5 mm apart and with the enzyme membranes prepared as in Examples 1 and 2 clipped to the surface and covering the first pair of electrodes (32, 34). A similar membrane, but without the enzyme is clipped across the pair of reference electrodes (36, 38). The probe was connected to a dual switching conductivity meter and immersed in 5 mM imidazole buffer pH 7.5. A base line was then obtained by commencing the measurement cycle on the instrument: for 15 seconds (variable) the instrument holds a zeroed value and then switching conductivity measurements are made for 45 seconds. The differential values (measured in mV) are stored and presented as a cumulative total at the end of the end of the 45 second period.

In the test procedure, various concentrations of the enzyme substrate are made up in 5 mM imidazole buffer pH 7.5 and held at a constant temperature of $25^{\circ}$C. (This is merely for experimental purposes, to eliminate changes in the conductivity due to temperature). After setting up the instrument base line using the straight 5 mM imidazole

16

buffer, the probe is removed from the buffer, wiped dry with tissue, and immersed in the sample to be tested. At the moment of immersion the measurement cycle is commenced, and at the end of 45 seconds the differential total ($\Delta$ mV) is displayed and recorded. After each measurement with a different sample the probe is wiped dry, washed with imidazole buffer and redried before use in the next sample.

The results obtained are tabulated below and illustrated graphically in Figures 10-15 of the accompanying drawings. These clearly demonstrate the close correlation between the differential conductivity of the two membranes measured as the differential output voltage ($\Delta$ mV) and the concentration of the enzyme substrate, and the feasibility, once the apparatus has been calibrated, or using a precalibrated membrane, of the method of the invention in the determination of an unknown concentration of the analyte in any given sample.

TABLE I

UREASE ON CELLULOSE NITRATE (Figs. 9 & 10)

| (Urea) mM | Mean $\Delta$ mV/ 45 second sampling period |
|-----------|---------------------------------------------|
| 0.5       | 41.00                                       |
| 1.0       | 84.25                                       |
| 2.0       | 170.00                                      |

| 3.5 | 276.25 |
|------|--------|
| 5.0 | 378.25 |
| 7.5 | 493.75 |
| 10.0 | 569.00 |

TABLE 2

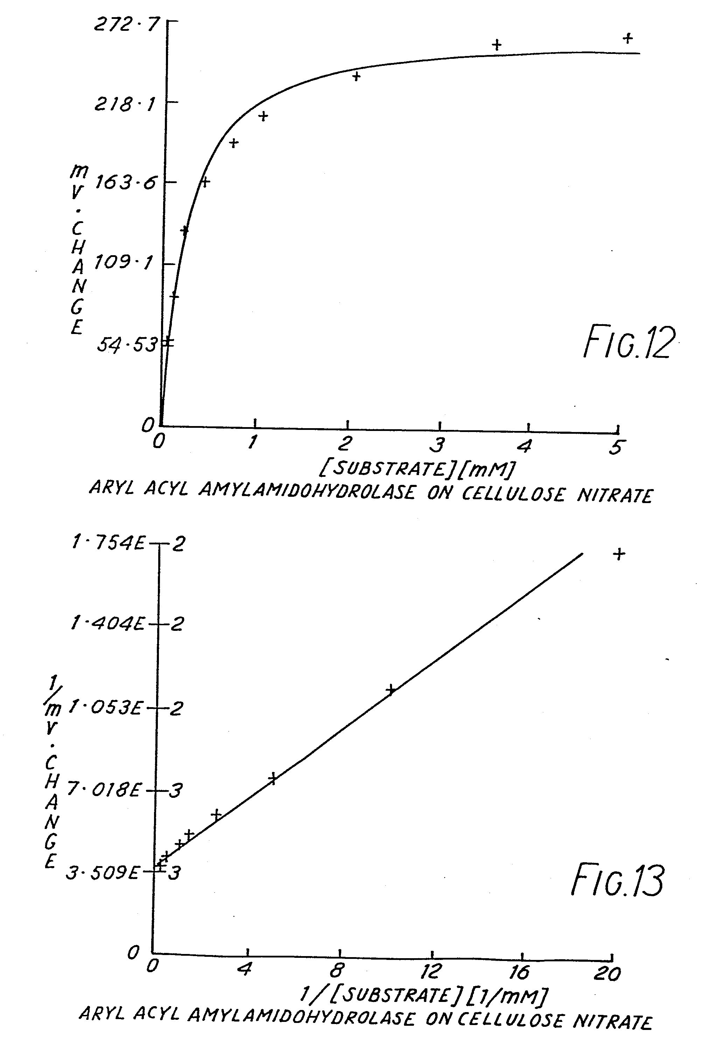

ARYL ACYL AMYLAMIDOHYDROLASE
ON CELLULOSE NITRATE (Figs. 11 & 12)

| (Paracetamol) mM | $\Delta$ mV/45 secs. |
|------------------|----------------------|
| 0.05 | 57.00 |
| 0.10 | 87.33 |
| 0.20 | 131.67 |
| 0.40 | 165.33 |
| 0.70 | 192.33 |

18

| 1.00 | 211.00 |
|------|--------|
| 2.00 | 239.75 |
| 3.50 | · 272.67 |
| 5.00 | .272.67 |

Km = 0.241 mM

V max = 274.3 mV

## TABLE 3

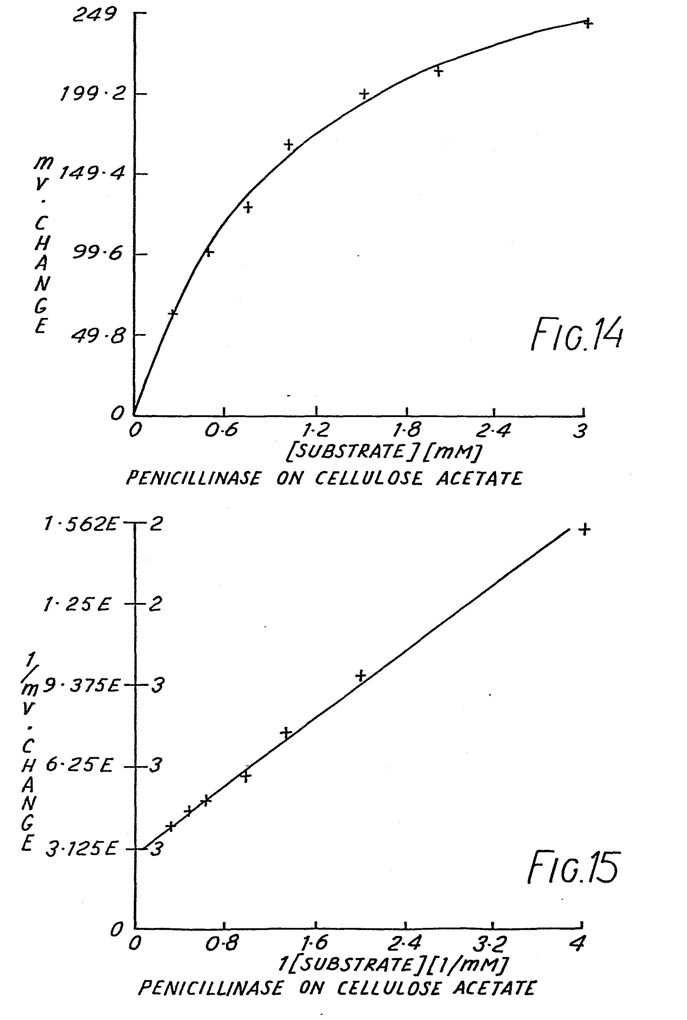

## PENICILLINASE ON CELLULOSE ACETATE (Figs. 13 & 14)

| (Ampicillin) mM | $\Delta$ mV/45 secs. |
|-----------------|----------------------|
| 0.25 | 64.00 |
| 0.50 | 101.67 · |
| 0.75 | 131.00 |
| 1.00 | 169.00 |
| 1.50 | 201.33 |

| | |
|---|---|
| 2.00 | 217.00 |
| 3.00 | 249.00 |

$Km = 1.13$ mM

$V$ max $= 344.8$ mV

20

## CLAIMS

1.      A conductimetric sensor for the quantitative bioassay of an analyte in a liquid sample comprising a sensing member whose conductivity, when in contact with the sample, is proportional to the concentration of the analyte, in the sample, and a pair of spaced-apart electrodes in electrical contact with the sensing member and connectable to means for measuring the conductivity of the sensing member, wherein said sensing member comprises an essentially non conducting porous membrane with which the electrodes are in face-to-face electrical contact at two separate points spaced along the length the membrane, said membrane being formed from a material having a conductivity of less than $10^{-9}$ Siemens/cm and having immobilised thereon a substance having a specific affinity for, or reactivity with the analyte, and reactive therewith in the presence of the sample to induce a change of conductivity along the length of the membrane between the two electrodes.

2.      A sensor according to Claim 1, wherein the two electrodes are in face-to-face contact with the same face of the membrane.

3.      A sensor according to Claim 1 or 2, wherein the membrane is cellulose acetate or cellulose nitrate.

4.      A sensor according to Claim 2 or 3, wherein the two electrodes are embedded in an elongated insulating support adjacent one end thereof, each electrode having an exposed surface substantially flush with the surface of the support and in electrical conatact with said membrane positioned or positionable on the surface of said support in overlapping relationship with the exposed electrode surfaces.

5.     A sensor according to Claim 4, wherein the membrane is carried by a slide member slidably and removably mounted on the elongated insulating support.

6.     A sensor according to Claim 5, wherein the membrane is exposed for contact with the liquid medium containing the analyte to be determined through an opening in the slide member.

7.     A sensor according to any one of Claims 1 to 6, wherein the substance immobilised onto the membrane is an enzyme.

8.     A sensor according to Claim 7, wherein the enzyme is a penicillinase, urease, glucose oxidase, creatinase, creatininase, paracetamolase, sarcosine hydrogenase, pronase or restriction endonuclease.

9.     A sensor according to any one of Claims 1 to 8, including a second pair of spaced apart reference electrodes and a second membrane positioned or positionable therebetween and in face-to-face contact therewith, said second membrane being substantially identical with the first membrane, and likewise introducable onto or contactable with said sample, but not having the said substance immobilised thereon, the said second membrane thus providing a reference value against which variations in the conductivity of the first membrane carrying the immobilised substance, when in contact with said sample, can be measured.

10.     A conductimetric bioassay apparatus comprising a sensor according to any one of Claims 1-9, and means for measuring the conductivity of the membrane(s) when in contact with the sample.

11.     Apparatus according to Claim 10, wherein the sensor is as claimed in Claim 9, and wherein the measuring means comprise means for alternately measuring the conductivity of the membrane carrying the immobilised substance and the conductivity of the reference membrane, means for comparing the two values and thereby to produce a differential value, means for storing and adding successive differential values obtained over a period of time, and means for displaying a cumulative total at the end of said period, such cumulative total thereby indicating the concentration of the analyte in the medium.

12.      A conductimetric bioassay method for the determination of an analyte in a liquid sample, which comprises contacting the sample with a sensor comprising an essentially non-conducting porous membrane formed from a material having a conductivity of less than $10^{-9}$ Siemens/cm and having immobilised thereon a substance having a specific affinity for a reactivity with the analyte, the interaction between the analyte and said substance in the presence of said sample being effective to cause a change in conductivity in the membrane, and measuring the change, if any, of conductivity of said membrane when in contact with said sample, said measurement being, made along the length of the membrane.

13.      A method according to Claim 12, wherein the membrane is of cellulose acetate or cellulose nitrate.

14.      A method according to Claim 12 or 13, wherein the analyte is an enzyme substrate and the immobilised substance is an enzyme having a specific activity for that substrate.

15.      A method according to Claim 14, wherein the analyte is a penicillin, glucose, urea, paracetamol, creatin, creatinin, sarcosine or nucleic acid, and the enzyme is, correspondingly, a penicillinase, glucose oxidase, urease, paracetamolase, creatinase, creatininase, sarcosine hydrogenase or restriction endonuclease.

16.      A method according to any one of Claims 12-15, wherein the membrane is contacted with the sample by placing a drop of the sample onto the membrane.

17.     A method according to any one of Claims 12-15, wherein the measurement is made by introducing into the liquid sample an elongated probe having a pair of spaced apart electrodes embedded in an insulating support member, the two electrodes being exposed at a surface of the support member, the support member carrying said membrane on its surface in face-to-face contact with said electrodes and forming a conductance member therebetween whose conductivity, in the presence of said sample, varies with the concentration of the analyte.

18.     A method according to any one of Claims 12-17, wherein the sample is simultaneously brought into contact with a second membrane substantially identical with the first membrane, but without any of said substance immobilised thereon, or on which the said substance has otherwise been inactivated, and the change of conductivity in the first membrane comprising the immobilised substance is compared to the conductivity in the second membrane.

19.     A method according to Claim 18, wherein the conductivity of the two membranes are measured alternately, and a final value displayed corresponding to the cumulative total of the successive differences in conductivity between the two membranes measured over a predetermined period of time.

20..     A method according to Claim 12, as applied to a competitive assay for the determination of a ligand or a specific binding partner of a ligand present in a sample in unknown quantity, which method comprises:

A)     contacting the sample with a porous membrane composed of a material having a conductivity of less than $10^{-9}$ Siemens/cm and having immobilized thereon a quantity of a specific binding partner of the ligand to be assayed, or of the ligand corresponding to the specific binding partner to be assayed, as the case may be, in the presence of a known quantity of the unknown ligand or specific binding partner, as the case may be, labelled with an enzyme, the quantity of one or other the immobilized component or the enzyme labelled component being restricted whereby competition takes place between the unknown and the enzyme labelled component for conjugation with the immobilized component;

B)    contacting the membrane obtained in step A and comprising an unknown quantity of the enzyme-labelled component conjugated to the immobilized component of the membrane with a substrate for the enzyme, the enzyme and substrate being such that operation of the enzyme on the substrate effects a change in conductivity of the conductance member constituted by the membrane and the immobilised enzyme-conjugate;

C)    measuring the change of conductivity of said conductance member, and

D)    determining therefrom the concentration of the unknown in the original sample.

21.    A conductimetric bioassay kit for the conductimetric assay of a ligand or a specific binding partner of a ligand in a sample, said kit comprising as a first item a porous membrane composed of a material having a conductivity of less than $10^{-9}$ Siemens/cm and having immobilized thereon a given quantity of a ligand or a specific binding partner of a ligand corresponding to the unknown, and as a second item, a given quantity of the unknown ligand or specific binding partner as the case may be, labelled with an enzyme, being such that when conjugated to the membrane by binding with the immobilized component, and contacted with an enzyme substrate, the conductivity of the membrane member formed by the membrane and the immobilised enzyme conjugate changes as a result of the enzyme/substrate interaction and proportional to the concentration of the unknown.

22.      A kit according to Claim 22, including, as a third item, a quantity of substrate for enzyme.

23.      A kit according to Claim 21 or 22, including, as a fourth item, means for measuring the electrical conductivity of the first item.

FIG.1

FIG.2

FIG.3

FIG.4

FIG.5

FIG.9

FIG.6

FIG.7

FIG.8

FIG.10

[SUBSTRATE] [mM]
UREASE ON CELLULOSE NITRATE

FIG.11

1/[SUBSTRATE] [1/mM]
UREASE ON CELLULOSE NITRATE

4/5

FIG.12

ARYL ACYL AMYLAMIDOHYDROLASE ON CELLULOSE NITRATE

FIG.13

ARYL ACYL AMYLAMIDOHYDROLASE ON CELLULOSE NITRATE

FIG.14

PENICILLINASE ON CELLULOSE ACETATE

FIG.15

PENICILLINASE ON CELLULOSE ACETATE

0121385

Application number

EP 84 30 1987

**European Patent Office**

**EUROPEAN SEARCH REPORT**

## DOCUMENTS CONSIDERED TO BE RELEVANT

| Category | Citation of document with indication. where appropriate, of relevant passages | Relevant to claim | CLASSIFICATION OF THE APPLICATION (Int. Cl. ³) |
|---|---|---|---|
| Y | GB-A-1 318 815 (MILES LAB.) <br><br> * Page 2, line 17 - page 4, line 12 * | 1,2,4, 7-12, 14,15, 17-21 | G 01 N 33/48 |
| | --- | | |
| P,Y | EP-A-0 096 095 (M. MALMROS) <br><br> * Page 5, line 25 - page 7, line 30 * | 1,6-8, 10,12, 14-16 | |
| | --- | | |
| A | EP-A-0 021 798 (EASTMAN KODAK) <br> * Frontpage * | 1 | |
| | ----- | | |
| | | | **TECHNICAL FIELDS SEARCHED (Int. Cl. ³)** |
| | | | G 01 N 33/48 |

The present search report has been drawn up for all claims

| Place of search | Date of completion of the search | Examiner |
|---|---|---|
| THE HAGUE | 03-07-1984 | DUCHATELLIER M.A. |